# EUROPEAN PATENT APPLICATION

(11) **EP 2 095 771 A1**
(43) Date of publication of application: **02.09.2009**
(21) Application number: 09002753.3
(22) Date of filing: 26.02.2009
(51) Int. Cl.: A61B 6/00, A61B 6/04, A61B 6/10

(54) **Radiation image information capturing apparatus**

(30) Priority: 29.02.2008 JP 2008051169
(71) Applicant: Fujifilm Corporation, Tokyo 106-8620 (JP)
(72) Inventor: Nakata, Hajime, Kanagawa 258-8538 (JP); Monma, Yoshiyuki, Kanagawa 258-8538 (JP)
(74) Representative: Klunker . Schmitt-Nilson . Hirsch

(57) **Abstract**

A mammography apparatus (10) is equipped with a radiation source housing unit (24) for housing a radiation source (23), which irradiates an examinee (22) with radiation, a radiographic image capturing base (26) that detects radiation having passed through the examinee (22) to acquire radiation image information, and a compression plate (28), which compresses and retains a region to be imaged of the examinee (22) with respect to the image capturing base (26). Pad members (46) are adhered detachably onto at least a front surface portion (36a) of an image capturing base (26), a front surface portion (40a) of a compression plate (28), and a face guard surface (42). The pad members (46) have a buffering property for absorbing shocks to the examinee (22), and a radiation shielding property for suppressing irradiation of X-rays with respect to the examinee (22).

## Description

### BACKGROUND OF THE INVENTION

### Field of the Invention:

The present invention relates to a radiation image information capturing apparatus equipped with a radiation source housing unit for housing a radiation source that irradiates an examinee with radiation, a radiographic image capturing base that detects radiation having passed through the examinee to acquire radiation image information, and a compression plate, which compresses and retains a region to be imaged of the examinee with respect to the image capturing base.

### Description of the Related Art:

In a radiation image information capturing apparatus, for example, in an X-ray mammography apparatus (hereinafter referred to simply as mammography apparatus), a radiation image of an object (breast of the examinee) is recorded in a radiation image recording unit (solid-state radiation detector) by irradiating the object with radiation, whereby reading of the radiation image information is performed.

As the aforementioned radiation image recording unit, normally, a solid-state radiation detection device of a light readout type, or of a type in which a plurality of photoelectric conversion elements and thin-film transistors (TFTs) are arranged, is used. In particular, recently, from an X-ray radiographic device utilizing an IP (imaging plate) cassette, in which a stimulable phosphor panel (IP) is accommodated, a cassetteless type of X-ray radiographic device has been developed, in which a digital image is acquired directly using a radiation conversion panel (flat panel detector, FPD).

However, in a mammography apparatus, in order to facilitate close contact of the examinee's breast against an image capturing base, the breast is compressed on the image capturing base using a compression plate. For this reason, there is a concern that the examinee may experience an unpleasant sensation of pressure accompanying positioning of the affected area to be imaged.

Consequently, for example, according to the specification of U.S. Patent No. 5,541,972, a padding device is disposed on a front surface and side surfaces of a plate portion of an X-ray plate (image capturing base), which comes into contact with the examinee's body. Further, according to the specifications of U.S. Patent No. 6,968,033 and U.S. Patent No. 6,765,984, a cushion is provided, which extends from a front surface across a pressing surface of the compression plate.

Furthermore, according to the specification of U.S. Patent No. 6,577,702, cushions are provided on surfaces of an image capturing base and a compression plate that come into contact with the examinee's body, whereas, according to the specification of U.S. Patent No. 6,850,590, buffering members are disposed on various surfaces including an image capturing surface of the image capturing base, which come into contact with the examinee's body.

However, according to the specification of U.S. Patent No. 5,541,972, in spite of reducing discomfort to the examinee when the examinee contacts a chest wall contacting portion of the image capturing base, there is still a concern that the examinee will be exposed to diffused X-rays, which pass from the image capturing base through the chest wall contacting portion.

Further, according to the specifications of U.S. Patent No. 6,968,033, U.S. Patent No. 6,765,984, U.S. Patent No. 6,577,702 and U.S. Patent No. 6,850,590, cushions (buffering members) are provided, which cover the X-ray irradiating region of the compression plate and the image capturing base. Consequently, for the cushions and the buffering members, materials that are highly permeable to X-rays must be used, and therefore it is easy for the examinee to be exposed to diffused X-rays.

### SUMMARY OF THE INVENTION

The present invention has the object of overcoming the problems mentioned above, by providing a radiation image information capturing apparatus, in which excessive pressure and discomfort to an examinee are suitably avoided, and wherein unnecessary exposure to diffused radiation with respect to the examinee can be suppressed.

The present invention relates to a radiation image information capturing apparatus, equipped with a radiation source housing unit for housing a radiation source, which irradiates an examinee with radiation, a radiographic image capturing base that detects radiation having passed through the examinee to acquire radiation image information, and a compression plate, which compresses and retains a region of the examinee to be imaged with respect to the image capturing base.

The radiation image information capturing apparatus further comprises a pad member positioned outside of an irradiation region extending from the radiation source to the image capturing base, and which is detachably disposed at least on a site having a possibility of coming into contact with the examinee. The pad member has a buffering property for absorbing shocks to the examinee, and a radiation shielding property for suppressing irradiation of radiation with respect to the examinee.

According to the present invention, the examinee does not come into contact directly with the outer walls of the radiation image information capturing apparatus, and due to the buffering effect of the pad members, shocks to the examinee can be absorbed and buffered. Owing thereto, the examinee does not experience a sensation of excessive pressure or discomfort, and pain caused by contact with the apparatus can suitably be avoided. In addition, the pad members have a radiation shielding property, so that unnecessary exposure of the examinee to diffused radiation can suitably be suppressed.

The above and other objects, features, and advantages of the present invention will become more apparent from the following description when taken in conjunction with the accompanying drawings in which preferred embodiments of the present invention are shown by way of illustrative example.

### BRIEF DESCRIPTION OF THE DRAWINGS

FIG. 1 is a perspective view of a mammography apparatus making up the radiation image information capturing apparatus according to a first embodiment of the present invention;
FIG. 2 is an explanatory drawing showing a portion of an image capturing base in the mammography apparatus;
FIG. 3 is a cross-sectional explanatory view of a pad member;
FIG. 4 is an explanatory drawing illustrating a cranio-caudal (CC) image capturing technique performed on an examinee;
FIG. 5 is an explanatory drawing showing positions on which pad members are adhered;
FIG. 6 is a perspective view of essential parts of a mammography apparatus according to a second embodiment of the present invention;
FIG. 7 is an explanatory drawing illustrating a medio-lateral oblique (MLO) image capturing technique performed on an examinee;
FIG. 8 is a perspective view of essential parts of a mammography apparatus according to a third embodiment of the present invention; and
FIG. 9 is a cross-sectional explanatory view of another pad member.

### DESCRIPTION OF THE PREFERRED EMBODIMENTS

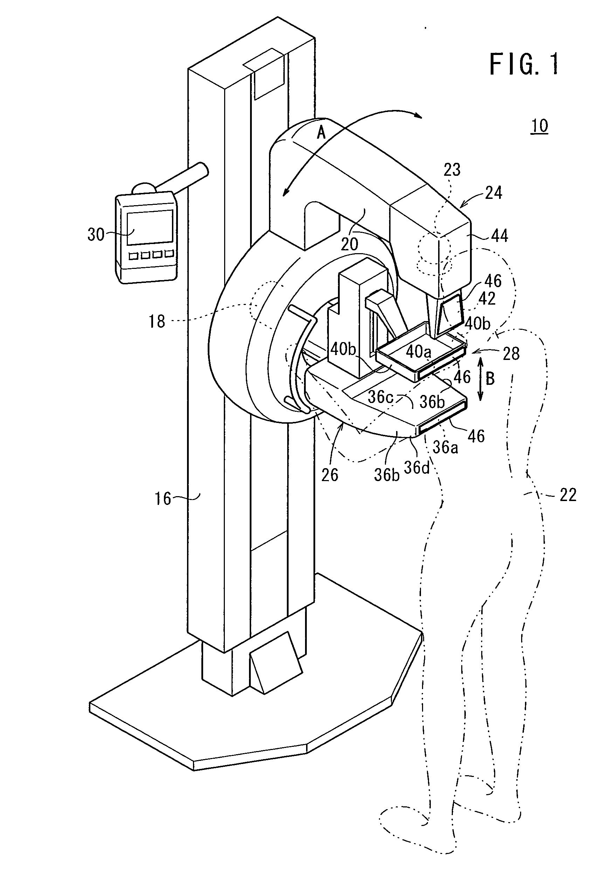

As shown in FIG. 1, a mammography apparatus 10 forming a radiation image information capturing apparatus according to a first embodiment of the present invention comprises a base 16 positioned in an upstanding manner, an arm member 20 fixed to a swing shaft 18 disposed substantially centrally on the base 16, a radiation source housing unit 24 fixed to an end of the arm member 20 and which accommodates therein an X-ray source (radiation source) 23 made up of a molybdenum tube, a tungsten tube, or a rhodium tube or the like, which irradiates X-rays (radiation) with respect to an examinee 22, an image capturing base 26 affixed to another end of the arm member 20 and housing therein a solid-state detector (not shown) for detecting radiation that has passed through the examinee 22, to thereby acquire radiation image information, and a compression plate 28 for compressing and holding a region to be imaged of the examinee 22 against the image capturing base 26.

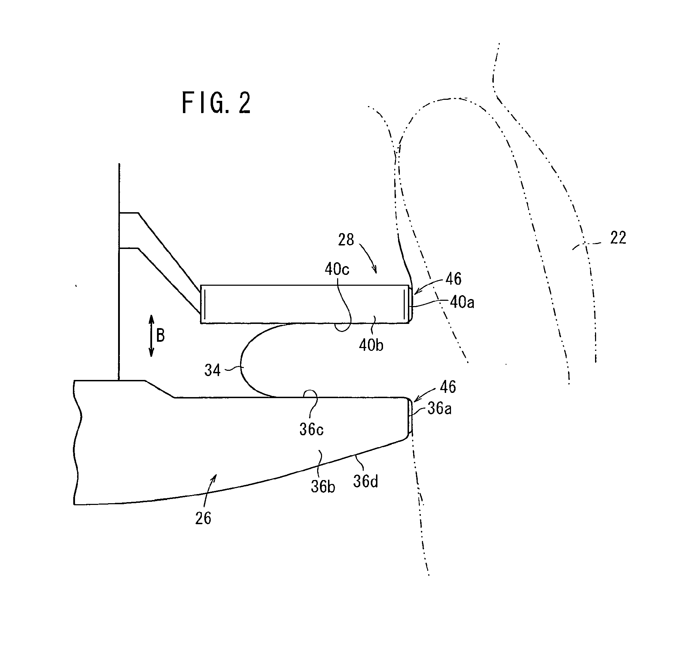

The solid-state detector comprises a direct-conversion, light readout type radiation solid-state detector. The solid-state detector stores radiation image information represented by X-ray radiation that has passed through a breast 34 (see FIG. 2) as an electrostatic latent image, and generates a current that depends on the electrostatic latent image when the solid-state detector is scanned by reading light.

The arm member 20, to which the radiation source housing unit 24 and the image capturing base 26 are secured, is constructed such that, by angularly moving the arm member 20 about a swing shaft 18 in the direction indicated by the arrow A, an image capturing direction with respect to the region to be imaged of the examinee 22 can be adjusted. The compression plate 28 is arranged between the radiation source housing unit 24 and the image capturing base 26, while being connected to the arm member 20. The compression plate 28 is configured for displacement in the direction of the arrow B.

A display control unit 30 is disposed on the base 16 for displaying image capturing information, including an image capturing region, an image capturing direction, and ID information of the examinee 22, etc., which have been detected by the mammography apparatus 10. The display control unit 30 also enables setting of such items of information as necessary.

FIG. 2 is an explanatory drawing showing a portion of the image capturing base 26 in the mammography apparatus 10, showing a condition in which a breast 34 as a region to be image of the examinee 22 is set between the image capturing base 26 and the compression plate 28.

As shown in FIGS. 1 and 2, the image capturing base 26 includes a front surface portion (chest wall portion) 36a facing toward the examinee 22, side surface portions (side wall portions) 36b, an image capturing surface 36c having an image capturing area 38, and a back surface portion 36d on an opposite side from the image capturing surface 36c. The compression plate 28 includes a front surface portion (chest wall portion) 40a facing toward the examinee 22, side surface portions (side wall portions) 40b on both sides thereof, and a pressing surface 40c confronting the image capturing surface 36c. The radiation source housing unit 24 has a face guard surface 42 that contacts the face of the examinee 22, and further comprises a casing (housing portion) outer surface 44 accommodating an X-ray source 23 therein.

In the first embodiment, pad members 46 are disposed detachably on a front surface portion 36a of the image capturing base 26, on a front surface portion 40a of the compression plate 28, and on a face guard surface 42 of the radiation source housing unit 24.

The pad members 46 have a buffering property for absorbing shocks to the examinee 22, as well as a radiation shielding property for suppressing radiation from being irradiated toward the examinee 22. More specifically, by forming the pad members 46 from a polymer material, for example, urethane foam, the pad members 46 function as a soft and flexible material having a buffering property, while functioning dually as a radiation shielding material with a low permeability to X-rays. Preferably, surfaces of the pad members 46 are such that skin oils (sebaceous matter) adhere only slightly thereto and do not lead significantly to any sensation of pulling on the skin, while also having an antibacterial property.

With respect to the X-rays irradiated from a molybdenum, tungsten, or rhodium target through a molybdenum, tungsten, rhodium or silver filter, the pad members 46 have such X-ray permeability that after having passed through the human body (examinee 22) and/or a member of the apparatus, the X rays are further attenuated to 50% or less. Therefore, the amount of diffused X-rays, which is irradiated onto the examinee 22, is suitably and significantly reduced.

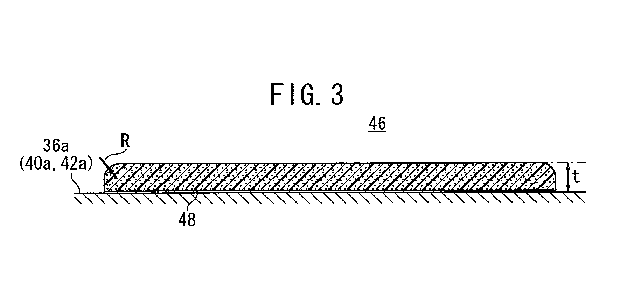

As shown in FIG. 3, the pad member 46 has an outer surface of a radius of curvature R of 1 mm or greater and a thickness t of 5 mm or less. Such a pad member 46 is attached (adhered) in a detachable manner, via a double-sided tape 48, with respect to the front surface portions 36a, 40a and the face guard surface 42.

Operations of the mammography apparatus shall now be explained.

First, using a console (not shown) and/or an ID card, etc., the operator sets ID information of the examinee 22, and image capturing information, such as various image capturing process details, etc. In this case, the ID information represents the name, age, sex, etc., of the examinee 22. Further, the image capturing process details include information such as an image capturing region, an image capturing direction, etc., which are specified by the doctor and may be entered by a technician from the console. The entered items of information can be displayed for confirmation on the display control unit 30 of the mammography apparatus 10.

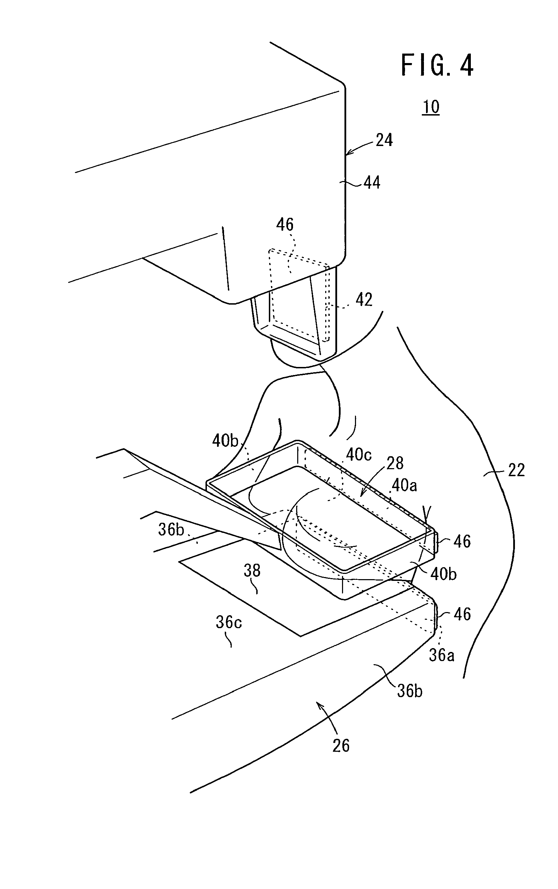

Then, the technician places the mammography apparatus 10 in a certain state in accordance with the specified image capturing method. For example, the breast 34 may be imaged as a cranio-caudal view (CC) in which an image is captured while X-rays are irradiated from above (see FIGS. 2 and 4), a medio-lateral view (ML) in which an image is captured while X-rays are irradiated from side surfaces outwardly from the center of the chest, or a medio-lateral oblique view (MLO) in which an image is captured while X-rays are irradiated from an oblique direction (to be described later). Depending on the selected image capturing method, the technician turns the arm member 20 about the swing shaft 18.

Next, the examinee 22 is placed in a specified image capturing condition with respect to the mammography apparatus 10. For example, if the left breast 34 of the examinee 22 is to be imaged as a cranio-caudal view (CC), as shown in FIGS. 2 and 4, the left breast 34 is positioned on the image capturing base 26, and thereafter, the compression plate 28 is lowered to hold the breast 34 between the image capturing base 26 and the compression plate 28.

Then, the X-ray source 23 accommodated in the radiation source housing unit 24 is energized in order to carry out capturing of the radiation image information. X-rays emitted from the radiation source, which have passed through the breast 34 held between the compression plate 28 and the image capturing base 26, are irradiated onto the solid-state detector (not shown) housed within the image capturing base 26, whereupon radiation image information of the breast 34 is acquired.

At this time, as shown in FIG. 4, because cranial-caudal (CC) imaging is performed with respect to the breast 34 of the examinee 22, the front surface portion 36a of the image capturing base 26 and the front surface portion 40a of the compression plate 28 are pressed against the examinee 22. In addition, the head of the examinee 22 presses against the face guard surface 42.

In this case, according to the first embodiment, as discussed above, when cranial-caudal (CC) imaging is performed with respect to the breast 34 of the examinee 22, pad members 46 are attached to the front surface portion 36a of the image capturing base 26, the front surface portion 40a of the compression plate 28, and the face guard surface 42, which come into direct contact with the examinee 22.

As a result, the front surface portions 36a, 40a and the face guard surface 42, which are constituted by a rigid carbon covering, do not directly contact the examinee 22, but rather, through the cushioning function of the pad members 46, any sensation of pressure experienced by the examinee 22 can suitably be alleviated. Owing thereto, the examinee does not experience a sensation of excessive pressure or discomfort when image capturing is performed, and pain caused by contact with the apparatus can suitably be avoided.

In addition, the pad members 46 are constructed from a polymer material such as urethane foam, which has an X-ray shielding property. As a result thereof, X-rays that are irradiated from the X-ray source 23 become diffused, for example, inside the image capturing base 26, and irradiation of such diffused X-rays toward the side of the examinee 22 from the front surface portion 36a can effectively be prevented by means of the pad member 46. Accordingly, unnecessary exposure to diffused X-ray radiation with respect to the examinee 22 can effectively be suppressed.

Similarly, due to the pad member 46 disposed on the front surface portion 40a of the compression plate 28, unnecessary exposure to diffused X-ray radiation with respect to the examinee 22 can be prevented. Also, due to the pad member 46 disposed on the face guard surface 42, unnecessary exposure to diffused X-ray radiation, directed from the face guard surface 42 toward the examinee 22, can suitably be suppressed.

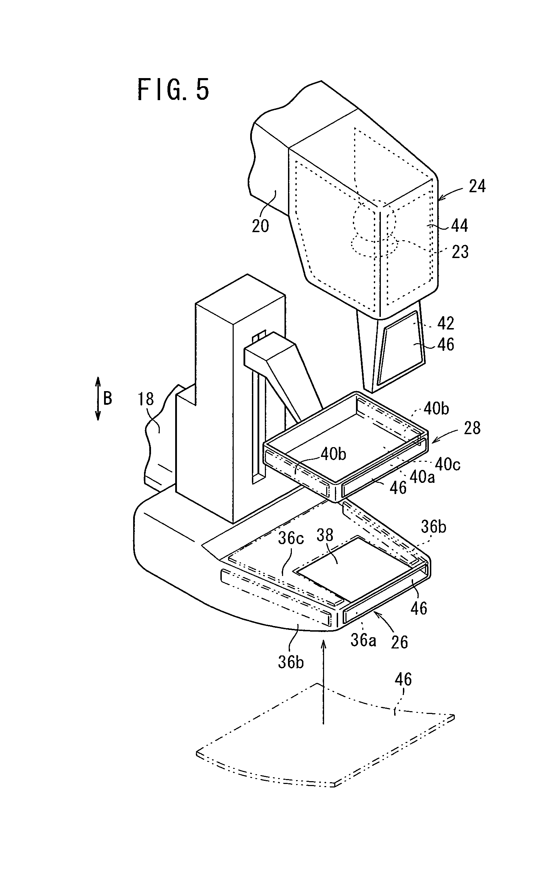

Further, as shown in FIG. 5, according to the first embodiment, the pad member 46 can be disposed on all or part of at least the front surface portion 36a of the image capturing base 26. Furthermore, on the image capturing base 26, apart from the front surface portion 36a, pad members can be disposed on at least any of all or part of each of the side surface portions 36b, on a region outside of the image capturing area (radiation irradiating area) 38 of the image capturing surface 36c, and on a back surface portion 36d of the image capturing base 26.

The pad members 46 are disposed on all or part of at least the front surface portion 40a of the compression plate 28. Pad members 46 can also be disposed on all or part of each of the side surface portions 40b of the compression plate 28.

On the other hand, on the radiation source housing unit 24, although the pad member 46 is disposed only on the face guard surface 42 thereof, if desired, pad members 46 may also be disposed from the front surface and over the side surfaces of the casing outer surface 44, which accommodate the X-ray source 23 therein. Owing thereto, when the arm member 20 is pivoted, a favorable condition results in that the casing outer surface 44 does not directly contact the examinee 22.

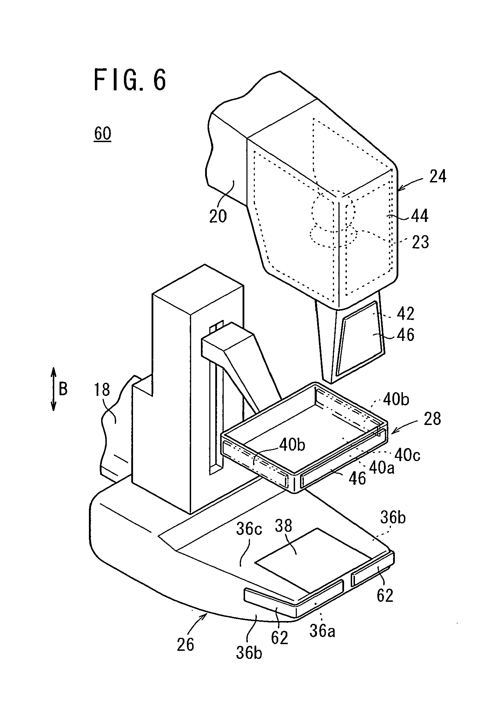

FIG. 6 is a perspective view of essential parts of a mammography apparatus 60 according to a second embodiment of the present invention. Structural elements thereof, which are the same as those of the mammography apparatus 10 according to the first embodiment, are designated with the same reference characters, and detailed explanations of such features are omitted. Further, similarly, in the third embodiment described below, detailed explanations of such features are omitted as well.

In the mammography apparatus 60, pad members 62 are adhered to the image capturing base 26, so as to cover the corners formed by the front surface portion 36a and both side surface portions 36b of the image capturing base 26. A single pad member 62 may be constructed as a unitary body. Alternatively, as shown in FIG. 6, the pad member 62 may be divided into two sections. The pad members 62 are constructed in the same manner as the above-mentioned pad member 46, having a desired buffering property for absorbing shocks and a desired X-ray radiation shielding property.

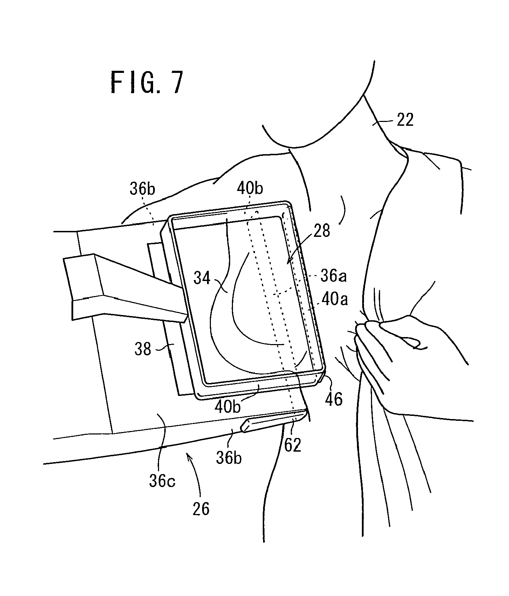

The mammography apparatus 60 is particularly suitable when capturing a medio-lateral oblique (MLO) image of the breast 34. More specifically, as shown in FIG. 7, when a medio-lateral oblique (MLO) image of the breast 34 of an examinee 22 is captured, the front surface portion 36a of the image capturing base 26, as well as both side surface portions 36b of the image capturing base 26 and the front surface portion 40a of the compression plate 28, come into contact with the examinee 22. Owing thereto, in particular, due to both front corner portions of the image capturing base 26, it is easy for a sense of pressure and discomfort to be imparted to the examinee 22.

Accordingly, by disposing the pad members 62 corresponding to the front corner portions of the image capturing base 26, any sensation of pressure or unpleasantness caused by the corners of the image capturing base 26 can suitably be suppressed.

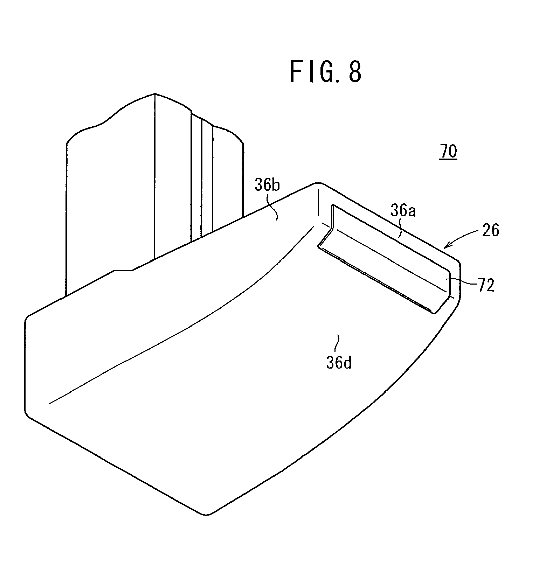

FIG. 8 is a perspective view of essential parts of a mammography apparatus 70 according to a third embodiment of the present invention.

A pad member 72 is attached to the image capturing base 26 that makes up the mammography apparatus 70, at a position corresponding to a corner area between the front surface portion 36a and the back surface portion 36d thereof. The pad member 72 produces the same effects as the aforementioned pad member 46.

As a result, according to the third embodiment, the corner area between the front surface portion 36a and the back surface portion 36d of the image capturing base 26 does not come into direct contact with the examinee 22. Owing thereto, any sensation of pressure or unpleasantness imposed on the examinee 22 can effectively be suppressed.

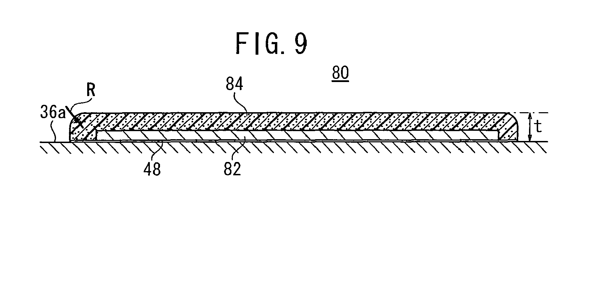

Moreover, in the first through third embodiments, pad members 46, 62 and 72 are used, which are constructed respectively in the same manner. However, in place of such a configuration, pad members 80 as shown in FIG. 9 may also be used.

The pad member 80, for example, includes a sheet member 82 with low radiation permeability, and a foam-containing soft flexible member 84, which are attached via a double-sided tape 48 to the front surface portion 36a. Specifically, the sheet member 82 may be formed by a lead sheet, a flame-retardant resin sheet, or the like, whereas the soft flexible member 84 is formed by a material having a desirable cushioning property. The pad member 80 configured in this manner produces the same effects as the aforementioned pad members 46, 62 and 72.

## Claims

1. A radiation image information capturing apparatus equipped with a radiation source housing unit (24) for housing a radiation source (23) that irradiates an examinee (22) with radiation, a radiographic image capturing base (26) that detects radiation having passed through the examinee (22) to acquire radiation image information, and a compression plate (28), which compresses and retains a region to be imaged of the examinee (22) with respect to the image capturing base (26), comprising:
a pad member (46) positioned outside of an irradiation region extending from the radiation source (23) to the image capturing base (26), and which is detachably disposed at least on a site having a possibility of coming into contact with the examinee (22),
wherein the pad member (46) has a buffering property for absorbing shocks to the examinee (22), and a radiation shielding property for suppressing irradiation of the radiation with respect to the examinee (22).

2. The radiation image information capturing apparatus according to claim 1, wherein the pad member (46) has an outer surface having a radius of curvature (R) of 1 mm or greater, and a thickness (t) of 5 mm or less.

3. The radiation image information capturing apparatus according to claim 1, wherein the pad member (46) is constituted by a material having a low permeability to radiation, or internally includes a material having a low permeability to radiation.

4. The radiation image information capturing apparatus according to claim 1, wherein with respect to X-rays irradiated from a molybdenum, tungsten, or rhodium target through a molybdenum, tungsten, rhodium or silver filter, the pad member (46) has a permeability to X-rays such that the X-rays after having passed through a human body and/or a member of the apparatus are further attenuated to 50% or less.

5. The radiation image information capturing apparatus according to claim 1, wherein the pad member (46) is disposed, with respect to the image capturing base (26), at least any of on all or part of a front surface portion (36a) thereof facing the examinee (22), on a region thereof outside of a radiation irradiating area (38) of an image capturing surface (36c), on all or part of side surface portions (36b) thereof, and on all or part of a back surface portion (36d) thereof opposite to the image capturing surface (36c).

6. The radiation image information capturing apparatus according to claim 1, wherein the pad member (46) is disposed, with respect to the compression plate (28), at least any of on all or part of a front surface portion (40a) thereof facing the examinee (22), and on all or part of side surface portions (40b) thereof.

7. The radiation image information capturing apparatus according to claim 1, wherein the pad member (46) is disposed, with respect to the radiation source housing unit (24), at least any of on all or part of a face guard surface (42) that contacts at least the face of the examinee (22), on all or part of a front surface of a housing portion (44) on the side of the examinee (22), and on all or part of side surfaces of the housing portion (44).
